# EUROPEAN PATENT APPLICATION

(11) **EP 4 684 796 A1**
(43) Date of publication of application: **28.01.2026**
(21) Application number: 23928887.1
(22) Date of filing: 01.12.2023
(51) Int. Cl.: A61K 39/145, A61P 31/16, A61K 39/00

(54) **INFLUENZA VACCINE COMPOSITION FOR PULMONARY DELIVERY**

(30) Priority: 23.03.2023 KR 20230038118
(71) Applicant: NA Vaccine Institute, Seoul 05854 (KR)
(72) Inventor: KIM, Dong Ho, Seongnam-si, Gyeonggi-do 13640 (KR); CHA, Seung Bin, Seoul 06584 (KR); KO, Kwang Hyun, Seoul 08792 (KR); BAE, Hyun Shik, Anyang-si, Gyeonggi-do 13944 (KR)
(74) Representative: Patentanwälte Olbricht Buchhold Keulertz
(86) International application number: PCT/KR2023/019718
(87) International publication number: WO 2024/195963

(57) **Abstract**

Provided is a pulmonary delivery vaccine composition for preventing or treating influenza virus infection, including a nucleic acid adjuvant and an influenza virus antigen.

## Description

### Technical Field

The present disclosure relates to a pulmonary delivery vaccine composition including a nucleic acid adjuvant and an influenza virus antigen.

### Background Art

Influenza vaccines must be administered annually due to antigenic drift and a duration of effectiveness of less than one year. The recommended vaccination period is set as October to December every year, taking into account the influenza epidemic period, i.e., December to April of the following year, and the duration of the vaccination effect, i.e., an average of 6 months.

Current influenza vaccines include inactivated vaccines and live attenuated influenza vaccines (LAIV). Inactivated vaccines are made by inactivating viruses cultured in fertilized eggs, i.e., embryonated eggs, using formalin or the like, or by culturing viruses in a cell culture system, inactivating them, harvesting the surface antigen, and administering it intramuscularly (i.m.) as a vaccine antigen.

Inactivated vaccines include whole virus vaccines that use the entire virus, split vaccines (subvirion) in which the viral envelope is removed using ether or the like, and subunit vaccines in which the hemagglutinin and neuraminidase components are purified. Hemagglutinin and neuraminidase are antigens that directly induce neutralizing antibody responses, and hemagglutinin is the main neutralizing antigen.

Antibodies obtained through natural infection or vaccination against a specific subtype or type of influenza virus generally do not form protective antibodies against other subtypes of influenza virus, and there are problems in that they also do not exhibit sufficient immunogenicity against new variants within a single antigen.

Because the influenza virus undergoes major and minor mutations every year, the prevalent strains change every year, and therefore it is difficult to expect protection from the vaccine administered the previous year, resulting in the difficulty of having to be vaccinated every year.

Meanwhile, adjuvants or immune potentiators are generally substances that are not sufficient in themselves to induce antibody production or cellular responses, but include single or mixed compounds or living agents that increase the immunogenicity of an antigen. Meanwhile, some adjuvants are also formulated to reduce immunogenicity and side effects. Overall, adjuvants provide a moderately potent and consistently robust antigen-specific enhanced immune response in a number of ways, including by facilitating antigen presentation to the immune system, possibly reducing antigen requirements, and possibly providing the additional benefit of avoiding multiple injections.

Innate immune cells recognize abnormal patterns or danger signals seen in invading infectious pathogens or vaccination antigens and transmit them to the adaptive immune system. Depending on the level and specificity of the pattern, it amplifies the qualitative and quantitative signals inherent in the adaptive response, demonstrating the importance of innate immunity in the adaptive immune response. Vaccines containing adjuvants are one of the most effective and cost-effective ways to prevent or treat disease.

Meanwhile, it is known that not only the humoral immune response through antibody production but also the cellular immune response through T cells plays an important role in preventing influenza virus infection. Since humoral immune responses cannot effectively remove antigens because antibodies cannot directly enter cells, cellular immune responses are needed to solve problems inside cells where antigens exist. That is, a cellular immune response is also required to more effectively remove antigens associated with the cell interior.

Meanwhile, vaccines administered intranasally work by penetrating the lower respiratory tract and inducing anti-hemagglutinin IgG antibodies. Both IgA, the key antibody of mucosal immunity, and serum IgG are involved in immunity against influenza virus. In particular, when mucosal immunity is stimulated through the nasal cavity, upper respiratory tract infections may be effectively prevented.

Respiratory IgA plays an important role in defense against influenza infection. An advantage of stimulating local respiratory IgA responses to influenza is that local respiratory IgA responses provide broader protective immunity than serum responses, and may provide cross-protection against viruses with hemagglutinin molecules that are different from the vaccine antigen. Therefore, an influenza vaccine that induces both anti-hemagglutinin responses in local secretory organs and serum would provide superior immunity to current vaccines. In contrast, parenteral vaccine injections (intramuscular, subcutaneous, etc.) are ineffective in inducing local antibody production unless there is separate mucosal exposure, i.e., infection. That is, in order to stimulate the immune system of the mucosa, the vaccine must be applied locally to the mucosal surface.

When an influenza vaccine is administered through the mucosa, such as by intranasal spray or dropwise administration, compared to traditional parenteral methods such as intramuscular, subcutaneous, or intravenous administration, the most notable advantages are that it more effectively stimulates the local mucosal immune system of the respiratory tract, and that it may increase the vaccination rate since it is free from rejection and anxiety related to needles.

In fact, when inactivated vaccines with low immunogenicity were administered intramucosally into humans, they induced stronger antibody responses and provided stronger protective immunity than when live or attenuated vaccines were administered intramuscularly. However, this mucosal administration method has not been commercially utilized due to the drawback that it requires approximately three times the amount used in intramuscular administration for patients.

To overcome the above problems, there have been other attempts to improve the immunogenicity of influenza vaccines when administered orally or intranasally, including the use of the cholera toxin B subunit (CTB), encapsulating the vaccine antigen in various microparticles, or using live attenuated strains.

To overcome the aforementioned limitations of conventional influenza vaccines, there has been a need for research on influenza vaccine compositions containing an adjuvant capable of inducing both humoral and cellular immune responses simultaneously, thereby providing sufficient cross-protective immunity. In addition, research has been needed on lung-delivered influenza vaccine compositions to overcome the limitations associated with mucosal administration methods of influenza vaccines.

### Disclosure

### Technical Problem

An aspect provides a pulmonary delivery vaccine composition for preventing or treating influenza virus infection including an influenza virus antigen as an active ingredient, wherein the influenza virus antigen includes hemagglutinin (HA) and nucleocapsid protein (NP), and the vaccine composition includes hetero structured RNA (hsRNA), and is delivered to the lungs.

Another aspect provides a universal influenza preventive vaccine including the pulmonary delivery vaccine composition, which induces both humoral and cellular immune responses simultaneously and has a cross-protection effect.

Another aspect provides a method of preventing or treating an influenza virus infection in a subject, including administering the pulmonary delivery vaccine composition for preventing or treating influenza virus infection to the subject.

### Technical Solution

An aspect provides a pulmonary delivery vaccine composition for preventing or treating influenza virus infection including an influenza virus antigen as an active ingredient, wherein the influenza virus antigen includes hemagglutinin (HA) and nucleocapsid protein (NP), and the vaccine composition includes hetero structured RNA (hsRNA), and is delivered to the lungs.

The influenza virus antigen may include hemagglutinin and nucleocapsid proteins.

The hemagglutinin protein functions to bind the virus to the host cell and is also involved in the release of new virus particles from the host cell. There are 18 types of hemagglutinin, and it is characterized as being the most variable among all influenza proteins.

In contrast, the nucleocapsid protein is a major structural protein found in influenza viruses, forms a shell around the viral RNA genome, is essential for proper assembly and release of new virus particles, and has very low variability. The nucleocapsid protein is composed of multiple copies of a single subunit and is found in the viral ribonucleoprotein (vRNP) complex and the viral core. Additionally, the vRNP complex provides a scaffold for the replication machinery and protects viral RNA from cellular degradation, and is therefore important for viral replication and transcription. Meanwhile, the nucleocapsid protein is also involved in the host immune response against the influenza virus in addition to its role in viral replication, and may stimulate the host immune system to recognize the virus and generate neutralizing antibodies. Antibodies generated against the nucleocapsid protein may provide long-lasting protection against future infections by the same viral strain.

Such nucleocapsid protein has highly conserved inter-subtype amino acid sequences capable of inducing broad spectrum immunity and may induce potent T cell immunity.

In the present disclosure, by using an antigen including hemagglutinin and nucleocapsid proteins and hetero structured RNA as an adjuvant, it is possible to provide an influenza virus pulmonary delivery vaccine composition capable of simultaneously inducing a neutralizing antibody response and a T cell immune response.

The influenza virus antigen may be one or more selected from the group consisting of influenza type A antigen, type B antigen, and type C antigen. For example, the influenza virus antigen may be one or more selected from the group consisting of type A antigen and type B antigen.

The influenza virus antigen may be an inactivated or live attenuated whole influenza virus, subvirion, or subunit.

The influenza virus antigen may be human influenza virus type A, type B, or type A and type B antigen. The human influenza virus type A, type B, or type A and type B antigen may be a whole influenza virus, a subvirion, or a partial subunit.

The human influenza antigen may be an inactivated or live attenuated whole influenza virus, or may be a part or whole of hemagglutinin and nucleocapsid proteins. The hemagglutinin or neuraminidase may be extracted from a whole virus propagation solution or may be expressed and purified by a recombinant method.

The vaccine composition may contain hetero structured RNA (hsRNA).

The hetero structured RNA may be a TLR 3 agonist. The hetero structured RNA may act as a TLR3 ligand.

As used herein, the term "TLR agonist" refers to an agonist that binds to and activates a toll-like receptor (TLR) expressed in a mammalian cell (e.g., a human cell). A TLR3 agonists bind to and activate TLR3.

As used herein, "hetero structured RNA (hsRNA)" refers to RNA having a structure in which a double-stranded RNA (dsRNA) region having complementarity of any length and any sequence is located, and a single-stranded RNA (ssRNA) is bound to both 3'-ends of the dsRNA. The ssRNA may act as a potential ligand for TLR7/8.

The hetero structured RNA may include a first strand including a 5'-end, a 3'-end and a region complementary to the second strand; a second strand including a 5'-end, a 3'-end and a region complementary to the first strand; a first overhang positioned at the 3'-end of the first strand having one or more consecutive ribonucleotides; and a second overhang positioned at the 3'-end of the second strand having one or more consecutive ribonucleotides. In the first strand and the second strand, the complementary region may be independently derived from a non-human subject.

The first and second overhangs may each have a UAUAG sequence. That is, the complementary regions may independently have a UAUAG sequence at the 3'-end.

TLR3 is normally expressed in endosomal compartments on dendritic cells (DCs), B cells, monocyte-derived macrophages, and many tumor tissues, and detects dsRNA. The dsRNA may be derived from viruses, bacteria, or abnormal cells.

When the TLR3 receptor recognizes dsRNA, it stimulates the secretion of type I interferon and proinflammatory cytokines. DCs are activated into mature antigenpresenting cells (APCs), and antigen epitopes are loaded onto MHC class I molecules and presented to naïve T cells. Activation of DCs by the TLR3 enzyme not only contributes to the induction of innate and adaptive immune responses against microbial pathogens, but also activates CD8+ T cells and natural killer (NK) cells. The minimum length of dsRNA capable of binding to TLR3 and inducing dimerization may be 45 mer.

The hetero structured RNA, when administered to a subject, may promote an immune response in the subject.

In the first strand and the second strand, the region complementary to the second strand may be complementary to the region complementary to the first strand. In the first strand and the second strand, the complementary region may have 75 % or more, 80 % or more, 85 % or more, 90 % or more, 95 % or more, or 100 % nucleotide sequence complementarity between the nucleotide sequences of the first strand and the second strand.

The complementary region may have a complementary nucleotide at every corresponding position between the nucleotide sequences of the first strand and the second strand.

In the first strand and the second strand, the complementary region may independently not include a sequence complementary to a nucleotide sequence of the human genome.

In the first strand and the second strand, the complementary region may be independently derived from a non-human subject. The subject may be derived from a virus, bacterium, or plant. The virus may be Sacbrood virus (SBC). The isolated double-stranded RNA molecule may be derived from the VP1 protein gene of Sacbrood virus.

In the first strand and the second strand, the first overhang and the second overhang may not include complementary sequences to each other. The first overhang and the second overhang may not contain complementary sequences to each other and thus may not form a double strand.

In the first strand and the second strand, the first overhang and the second overhang may be independently obtained by cleavage of an RNA molecule including a double-stranded region and a single-stranded region by using an RNase, for example, RNase T1. In the first strand and the second strand, the first overhang and the second overhang may be independently obtained by hybridizing a pre-first strand having nucleotides extended at the 3'-end compared to the first strand, and a pre-second strand having nucleotides extended at the 3'-end compared to the second strand, and contacting the hybridized product with an RNase, for example, RNase T1, to digest ssRNA at the 3'-end.

The RNase may be an endoribonuclease or exoribonuclease that specifically cleaves single-stranded RNA. The RNase T1 may be an endoribonuclease that specifically cleaves single-stranded RNA at G residues. The RNase T1 may cleave the phosphodiester bond between the 3'-guanyl residue and the 5'-OH residue of the neighboring nucleotide. The reaction product may be a polynucleotide having 3'-GMP and a terminal 3'-GMP. The reaction may be performed under conditions that allow RNase T1 to specifically cleave single-stranded RNA at G residues for the RNA substrate.

The first strand and the second strand may respectively have the nucleotide sequences of SEQ ID NO: 1 and SEQ ID NO: 2.

In the first strand and the second strand, the 5'-end may independently not have a 5'-cap.

In the first strand and the second strand, the 5'-end may independently have a triphosphate, a diphosphate, or a monophosphate.

In the first strand and the second strand, the 3'-end may independently have a phosphate.

In the first strand and the second strand, the 3'-end may independently have a hydroxyl group.

By using an influenza antigen including hemagglutinin and nucleocapsid protein and the hetero structured RNA, the composition may be capable of simultaneously inducing a neutralizing antibody response and a T cell immune response.

The T cell immune response may be a CD4⁺ cell immune response.

The pulmonary delivery vaccine composition, by using hetero structured RNA as an adjuvant, may induce type I interferon (type I IFN) in cells, for example, in immune cells including dendritic cells, by the hetero structured RNA delivered to the lungs, and may form CD4⁺ Trm (tissue-resident memory T) cells in the lungs.

The vaccine composition for preventing or treating influenza virus infection may be administered through the respiratory tract and delivered to the lungs.

The term "lung" refers to the organs and tissues of the lower respiratory tract, examples of which may include, but are not limited to, the lungs including their lobes, tips, lingulae, and alveoli; the bronchi including respiratory bronchioles; the tracheal and bronchial rings including the carina; the pulmonary blood vessels including the pulmonary vessels and bronchial blood vessels and bronchial blood vessels; the bronchopulmonary lymph nodes; and the autonomic nervous system of the lung. The deep lungs or alveoli are the primary targets of inhaled therapeutic aerosols for systemic drug delivery.

The administration of the pulmonary delivery vaccine composition of the present disclosure may be accomplished by any route of administration known to those skilled in the art. The route of administration is not limited as long as it is for pulmonary delivery, and for example, may be administered via the respiratory system as a route for delivery to the lungs.

The respiratory system is a comprehensive term that includes the upper and lower respiratory tracts. The upper respiratory tract includes the nasal cavity, oral cavity, and pharynx/larynx. The lower respiratory tract includes all structures below the trachea.

The vaccine composition may be administered by dropwise application to the respiratory tract.

The vaccine composition may have a formulation suitable for administration through the respiratory tract. The formulation may be a liquid or a spray formulation.

When the vaccine composition is administered via the respiratory tract, the hetero structured RNA must reach the lungs in order to form CD4+ Trm (tissue-resident memory T) cells in the lung. Since the vaccine composition must reach the lungs of the subject, in the case of respiratory administration, for example, administration by inhalation, the particle size of the composition may be sufficiently small to reach the distal part of the airways of the lung tissue.

The vaccine composition may be composed of particles that are effective in penetrating into the alveoli of the lungs, i.e., particles having a mass median aerodynamic diameter (MMAD) of about 0.1 µm to about 10 µm. The particle diameter is 10 µm or less, for example, 5 µm or less, and typically may be within the range of 0.1 µm to 5 µm aerodynamic diameter depending on the specific properties of the particle. Such particles may be effective in targeting the lungs when administered by inhalation.

The optimal inhalation device for inhalation application of the pharmaceutically active agent is not particularly limited, but a nebulizer known in the art may be used.

Nebulizers are used to administer active ingredients in the form of a mist that is inhaled into the lungs. Physically, such mists are aerosols based on liquid droplets. This is produced within the nebulizer by splitting solutions and suspensions into small aerosol droplets that may be inhaled directly from the mouthpiece of the device. In conventional nebulizers, the aerosol may be produced by mechanical force, such as spring force within a jet nebulizer, or by electrical force. In a jet nebulizer, a compressor forces oxygen or compressed air at high velocity through an aqueous solution containing the active ingredient, which creates an aerosol. An ultrasonic nebulizer uses an electronic oscillator that causes vibration of a piezoelectric element to generate ultrasonic waves in a liquid reservoir containing the active ingredient at high frequency.

In an embodiment of the present disclosure, the vaccine composition may be aerosolized and delivered to the patient's lungs through inhalation by the patient. Pulmonary delivery may have advantages over systemic administration. The amount of active agent that may be delivered to the lungs via non-pulmonary routes, such as systemic administration, may be limited to the minimum pharmacologically effective dose. By administering the active agent directly to the lungs by inhalation, greater amounts may be delivered to the area requiring treatment, substantially reducing systemic exposure.

The pulmonary delivery vaccine composition of the present disclosure for preventing or treating influenza virus infection may have a cross-protection effect.

The pulmonary delivery vaccine composition may provide heterosubtypic immunity against influenza virus and homosubtypic immunity against influenza virus.

The heterosubtypic immunity refers to protection against a different subtype or strain of influenza virus despite being immunized against one subtype or strain of influenza virus. For example, it refers to immunity that allows protection against a different subtype of influenza A virus to which the individual has not been previously exposed, even though the person has been vaccinated against one subtype of influenza A virus.

The homosubtypic immunity refers to protection against the same subtype or strain of the influenza virus, and also includes protection against other strains of the same subtype. For example, it refers to immunity that allows protection against a different strain of influenza A (H1N1) virus to which the individual has not been previously exposed, even though the person has been vaccinated against one strain of influenza A (H1N1) virus.

The homosubtypic immunity may also be applied even when the host species is different.

The pulmonary delivery vaccine composition for preventing or treating influenza virus infection of the present disclosure, by using hetero structured RNA as an adjuvant, may induce type I interferon (IFN) by the hetero structured RNA delivered to the lungs, and form CD4+ Trm cells (tissue-resident memory T cells) in the lungs, thereby providing not only homotypic immunity but also heterotypic immunity against influenza virus.

Another aspect provides an influenza universal preventive vaccine including the pulmonary delivery vaccine composition, which induces both humoral and cellular immune responses simultaneously and has a cross-protection effect.

Another aspect provides a method of preventing or treating an influenza virus infection in a subject, including administering the pulmonary delivery vaccine composition for preventing or treating an influenza virus infection to the subject.

The administration may be in an effective amount for preventing or treating influenza virus infection in the subject. The term "effective amount" may be selected by a person skilled in the art depending on factors such as the weight and sex of the subject. The administration may be administered orally or parenterally. The administration may be via a mucosa, for example, the nasal mucosa. Additionally, the administration may be by inhalation through the nose or mouth.

The subject may be a mammal, for example a human, a dog, a cat, a cow, a pig or a sheep. The subject may be an individual not infected with the influenza virus. Additionally, the subject may be an individual infected with an influenza virus.

### Advantageous Effects

The pulmonary delivery vaccine composition for preventing or treating influenza virus infection according to an aspect may simultaneously induce a neutralizing antibody response and a T cell immune response by using an influenza antigen including hemagglutinin and nucleocapsid proteins, and hetero structured RNA.

In addition, the pulmonary delivery vaccine composition, by using hetero structured RNA as an adjuvant, may induce type I interferon (IFN) by the hetero structured RNA delivered to the lungs, and form CD4+ Trm (tissue-resident memory T) cells in the lungs, thereby providing not only homosubtypic immunity but also heterosubtypic immunity against the influenza virus.

The universal influenza preventive vaccine including the pulmonary delivery vaccine composition according to another aspect may have a cross-protection effect by simultaneously inducing humoral and cellular immune responses.

### Description of Drawings

FIG. 1A is a diagram showing the experimental method of Example 1.
FIG. 1B is a diagram showing the results of measuring levels of antigen-specific IgM, IgG, IgG1, IgG2, and IgG3 antibodies in serum after intramuscular and intranasal dropwise administration, respectively, of a vaccine including Teratect prefilled syringe (Influenza split vaccine), or NVT and Teratect prefilled syringe (Influenza split vaccine).
FIG. 1C is a diagram showing the results of measuring IgA levels in BALF, nasal wash, and serum after intramuscular and intranasal dropwise administration, respectively, of a vaccine including Teratect prefilled syringe (Influenza split vaccine), or NVT and Teratect prefilled syringe (Influenza split vaccine).
FIG. 1D is a diagram showing the results of measuring antigen-specific T cell responses in the lungs and spleen by using an intracellular cytokine staining method after intramuscular and intranasal dropwise administration, respectively, of a vaccine containing Teratect prefilled syringe (Influenza split vaccine), or NVT and Teratect prefilled syringe (Influenza split vaccine).
FIG. 1E is a diagram showing the results of measuring body weight according to the date of intramuscular and intranasal dropwise administration, respectively, of vaccines including Teratect prefilled syringe (Influenza split vaccine), or NVT and Teratect prefilled syringe (Influenza split vaccine), respectively.
FIG. 2A is a diagram showing the experimental method of Example 2.
FIG. 2B shows the results of measuring neutralizing antibody responses against H3N2, the same subtype as the vaccine antigen, and H1N1, a different subtype, in serum, BALF, and nasal wash fluid after intramuscular or intranasal dropwise administration of a vaccine including H3N2 antigen, or NVT and H3N2 antigen, using a hemagglutination inhibition assay.
FIG. 2C is a diagram showing the results of measuring antigen-specific T cell responses in the lung and spleen against H3N2, the same subtype as the vaccine antigen, and H1N1, a different subtype, after intramuscular or intranasal dropwise administration of a vaccine including H3N2 antigen, or NVT and H3N2 antigen, using intracellular cytokine staining.
FIG. 2D is a diagram showing the number of CD4⁺ T cells and CD8⁺ T cells observed in BALF after intramuscular or intranasal dropwise administration of a vaccine including H3N2 antigen, or NVT and H3N2 antigen.
FIG. 2E is a diagram showing changes in body weight and survival rate after challenge with H1N1 virus, a different subtype from the vaccine antigen, in mice that received intramuscular or intranasal dropwise administration of a vaccine including H3N2 antigen, or NVT and H3N2 antigen.
FIG. 2F is a diagram showing viral titers in the lungs on day 3 and day 6 after challenge with H1N1 virus, a different subtype from the vaccine antigen, in mice that received intramuscular or intranasal dropwise administration of a vaccine including H3N2 antigen, or NVT and H3N2 antigen.
FIG. 2G is a diagram showing the results of histopathological examination of the lungs on day 6 after challenge with H1N1 virus, a different subtype from the vaccine antigen, in mice that received intramuscular or intranasal dropwise administration of a vaccine including H3N2 antigen, or NVT and H3N2 antigen.
FIG. 3A is a diagram showing the experimental method of Example 3.
FIG. 3B is a diagram showing the results of measuring the number of CD4⁺ T cells in the lung and spleen in a group administered a CD4 depletion antibody after receiving H3N2 intramuscular vaccine or NVT + H3N2 intranasal vaccine, and in an experimental group administered an isotype antibody after receiving NVT + H3N2 intranasal vaccine.
FIG. 3C is a diagram showing the results of measuring neutralizing antibody titers against H1N1, a different subtype from the vaccine antigen, in the serum of a group administered a CD4 depletion antibody after receiving H3N2 intramuscular vaccine or NVT + H3N2 intranasal vaccine, and an experimental group administered an isotype antibody after receiving NVT + H3N2 intranasal vaccine.
FIG. 3D is a diagram showing the results of measuring antigen-specific IgA titers in nasal wash fluid against H3N2, the same subtype as the vaccine antigen, and H1N1, a different subtype, in a group administered a CD4 depletion antibody after receiving H3N2 intramuscular vaccine or NVT + H3N2 intranasal vaccine, and an experimental group administered an isotype antibody after receiving NVT + H3N2 intranasal vaccine.
FIG. 3E is a diagram showing changes in body weight and survival rate over time after H1N1 virus challenge in mice administered an H3N2 intramuscular vaccine or an NVT and H3N2 antigen-containing vaccine intranasally, with or without CD4 T cell depletion.
FIG. 4A is a drawing showing the experimental method of Example 4.
FIG. 4B is a diagram comparing antigen-specific T cell responses in the lung and spleen following administration of H3N2 intramuscular vaccine, NVT + H3N2 intranasal vaccine, or NVT + H3N2 intramuscular vaccine.
FIG. 4C is a diagram comparing T cell subsets in the lung following administration of H3N2 intramuscular vaccine, NVT + H3N2 intranasal vaccine, or NVT + H3N2 intramuscular vaccine.
FIG. 4D is a diagram comparing the proportion and number of resident memory T cell subsets in the lung following administration of H3N2 intramuscular vaccine, NVT + H3N2 intranasal vaccine, or NVT + H3N2 intramuscular vaccine.
FIG. 4E is a diagram showing changes in body weight and survival rate over time after challenge with H1N1 virus in mice administered an H3N2 intramuscular vaccine, an NVT + H3N2 intranasal vaccine, or an NVT + H3N2 intramuscular vaccine.
FIG. 5A is a diagram showing the experimental method of Example 5.
FIG. 5B is a diagram comparing T cell subsets in the lung when an NVT + H3N2 intranasal vaccine was administered to wild-type (WT) mice and knockout (KO) mice, respectively.
FIG. 5C is a diagram comparing the proportion and number of resident memory T cell subsets in the lung when NVT + H3N2 intranasal vaccine was administered to wild-type (WT) mice and knockout (KO) mice, respectively.
FIG. 5D is a diagram comparing antigen-specific T cell responses in the lung and spleen when an NVT + H3N2 intranasal vaccine was administered to wild-type (WT) mice and knockout (KO) mice, respectively.
FIG. 5E is a diagram comparing the titers of antigen-specific IgG and IgA in serum and nasal wash fluid when NVT + H3N2 intranasal vaccine was administered to wild-type (WT) mice and knockout (KO) mice, respectively.
FIG. 6A is graphs showing changes in survival rate and body weight when a quadrivalent vaccine (QIV) containing the H1N1 pdm09 strain was administered twice intramuscularly at two-week intervals or intranasally in combination with NVT, followed by challenge with the H1N1 non-pdm09 strain influenza A/PR8/34 (H1N1).
FIG. 6B is graphs showing changes in body weight and survival rate when a human H3N2 vaccine was administered twice intramuscularly at two-week intervals or intranasally in combination with NVT, followed by challenge with a canine H3N2 strain.
FIG. 6C is graphs showing the measurement results of CD4+ IFNv+ T cells when stimulated with BV virus or BY virus, comparing intranasal dropwise administration of inactivated BV and NVT-containing vaccine, and inactivated BY and NVT-containing vaccine, with intramuscular administration of inactivated BV-containing vaccine and inactivated BY-containing vaccine.
FIG. 6D is a diagram showing changes in body weight over time after intramuscular or intranasal dropwise administration of vaccines containing BV antigen, BY antigen, or NVT and BY antigen, and the survival rate after challenge with a different subtype BV virus.
FIG. 7A is a diagram showing the results of measuring HAI titers after intramuscular or intranasal dropwise administration of Teratect prefilled syringe (Influenza split vaccine), or a vaccine comprising NVT and Teratect prefilled syringe (Influenza split vaccine).
FIG. 7B is a diagram showing the results of measuring CD4+ IFNv+ T cell levels in the lung after intramuscular or intranasal dropwise administration of Teratect prefilled syringe (Influenza split vaccine), or a vaccine comprising NVT and Teratect prefilled syringe (Influenza split vaccine).
FIG. 8A shows photographs comparing the extent of pulmonary delivery by isolating the lungs after dropwise administration of 10 µL Evans Blue solution (left) and 30 µL Evans Blue solution (right).
FIG. 8B is a graph confirming that a strong cellular immune response in the lung was observed when a drug was delivered to the lungs.

### Best Mode

Hereinafter, the present disclosure will be described in more detail through examples. However, these examples are intended to exemplify the present disclosure and the scope of the present disclosure is not limited to these examples.

### Preparation Example: Preparation of Hetero Structured RNA and Vaccine Composition

DNA fragments were synthesized in which a sequence including the T7 promoter sequence (SEQ ID NO: 3) was linked to the 5'-end of DNA sequences encoding the nucleotide sequences of SEQ ID NO: 1 and SEQ ID NO: 2. The DNA fragment was a dsDNA formed from the nucleotide sequence of SEQ ID NO: 4 and its complementary sequence. The DNA fragment was ligated into a PUC19 vector (Thermo Fisher, Cat SD0061) digested with Smal to prepare a recombinant vector.

Next, the recombinant vector was introduced into E. coli DH5α by transformation. The obtained recombinant E. coli were cultured in L7B/amp medium to proliferate the E. coli containing the recombinant vector. The proliferated E. coli in the culture were separated from the supernatant, and the recombinant vector was isolated from the E. coli using an alkaline lysis method (Qiagen midi prep kit). The template DNA for in vitro transcription (IVT) based on T7 polymerase was amplified by PCR using the recombinant vector DNA as a template. At this time, oligonucleotides having the nucleotide sequences of SEQ ID NO: 5 and SEQ ID NO: 6, corresponding to both ends of the target sequence, were used as the primer set.

The PCR conditions are as follows. After one cycle at 95 °C for 5 minutes, thermal cycling was performed for 35 cycles at 95 °C for 30 seconds, 60 °C for 30 seconds, and 72 °C for 1 minute. Finally, the reaction was held at 72 °C for 5 minutes to complete the PCR reaction. The amplified DNA product was used as a template to perform an in vitro transcription (IVT) reaction using the MEGAscript^{™} T7 Transcription Kit (Thermo Fisher, Cat AMB13345). In a 1 ml reaction solution, 10 µg of linear template DNA, 75 mM NTP, 90 mM Tris base, 90 mM boric acid, 2 mM EDTA, and 50 µL of T7 polymerase were added and reacted at 37°C for 4 hours. After the reaction, the mixture was heat-treated at 80 °C for 20 minutes and then cooled at room temperature for 30 minutes. Through the IVT, the first strand and the second strand were simultaneously synthesized from both strands and hybridized to form the hetero structured RNA.

The cooled reaction mixture was centrifuged at 4000 rpm at 20 °C for 3 minutes using a centrifuge to remove the white precipitate and obtain the supernatant. DNase I (1/6500 dilution from stock) was added to the obtained supernatant and reacted at 37 °C for 13 hours. Afterwards, RNase T1 diluted 1/20,000 was added to the reaction mixture and reacted at 37 °C for 2 hours. Afterwards, the reaction mixture was incubated at 80 °C for 10 minutes and then cooled to room temperature for 30 minutes. Next, using a nucleic acid precipitation method with isopropanol, hetero structured RNA having a 3'-overhang at each 3'-end was isolated from the reaction mixture. The obtained hetero structured RNA having the 3'-overhang is hereinafter referred to as "NVT".

The hetero structured RNA may have a phosphate at the 5'-end and a hydroxyl group at the 3'-end. The phosphate may be a triphosphate, a diphosphate, or a monophosphate. The hetero structured RNA may not have a 5'-cap. The hetero structured RNA may have a triphosphate at the 5'-end and a hydroxyl group at the 3'-end.

The NVT was dissolved in inactivated quadrivalent influenza virus vaccine (QIV) antigen or H3N2 monovalent antigen in PBS (pH 7.2) to prepare a vaccine composition containing hetero structured RNA (NVT). At this time, the total amount of antigen and NVT were mixed at a weight ratio of 1:1 to 1:10. The total amount of QIV antigen was adjusted to be 1 µg to 10 µg per mouse. The prepared vaccine composition was stored at 4 °C to 8 °C. In the following examples, a total of 30 µL of the vaccine composition was administered dropwise into each nostril of the mouse, divided equally.

### Example 1: Determination of Overall Immunological Profile after Pulmonary Delivery

It was confirmed whether intranasal administration shows superior protective efficacy compared to intramuscular injection during actual viral infection. The commercially available quadrivalent vaccine, Teratect prefilled syringe (Influenza split vaccine) (Ilyang Pharmaceutical Co. Ltd.)was administered intramuscularly to a subject, or a vaccine mixed with NVT was prepared and administered intranasally by dropwise application to the subject. Mice were used as the subjects. The Teratect prefilled syringe (Influenza split vaccine) is a quadrivalent antigen vaccine containing antigens from influenza A H1N1, specifically A/Victoria/2570/2019, IVR-215 (H1N1); A H3N2, specifically A/Cambodia/E0826360/2020, IVR-224 (H3N2); influenza B BV, specifically B/Maryland/15/2016; and BY, specifically B/Phuket/3073/2013. The Teratect prefilled syringe vaccine is a split vaccine containing HA and NP antigens.

As a control group, PBS was administered either intramuscularly (i.m.) or intranasally (i.n.).

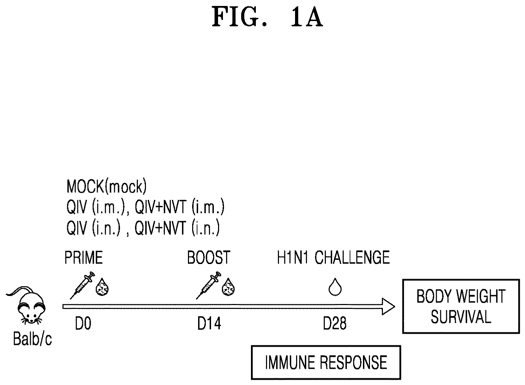

As shown in FIG. 1A, the vaccine was administered intranasally to mice by dropwise application, and two weeks later, a booster dose was administered using the same method. Two weeks after the booster, BALF, nasal wash fluid, and serum were collected. Using the ELISA method, the levels of antigen-specific IgM, IgG, IgG1, IgG2, and IgG3 antibodies in the serum were measured, and the levels of antigen-specific IgA antibodies in the BALF and nasal wash fluid were measured. In addition, antigen-specific T cell responses in the lung and spleen were measured using intracellular cytokine staining. In some mice, two weeks after the booster administration, challenge was performed by intranasal dropwise administration of 3LD50 of the H1N1 virus, which is the subtype included in the administered vaccine.

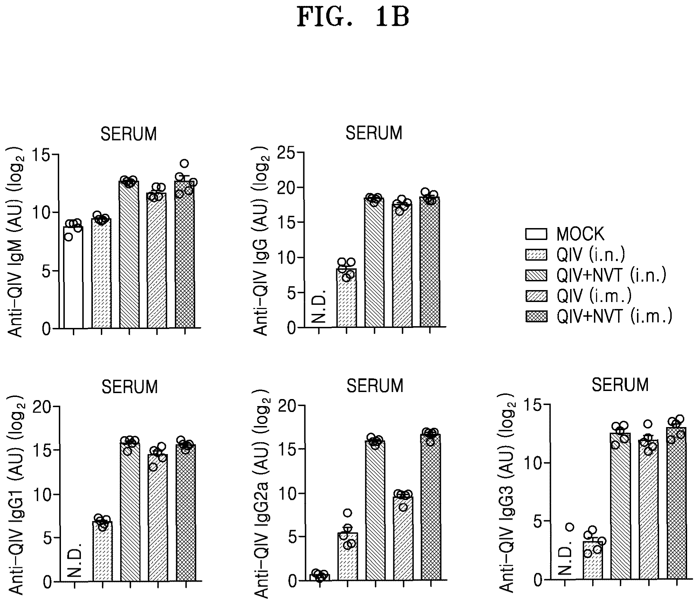

FIG. 1B is a diagram showing the results of measuring the levels of antigen-specific IgM, IgG, IgG1, IgG2, and IgG3 antibodies in the serum after intramuscular and intranasal dropwise administration, respectively, of a vaccine including Teratect prefilled syringe (Influenza split vaccine), or NVT and Teratect prefilled syringe (Influenza split vaccine). As shown in FIG. 1B, IgG, IgG1, IgG2, and IgG3 levels in the serum were higher compared to the control group. However, a similar level of IgG was also observed after intramuscular administration, indicating that the IgG response observed in serum does not show a significant difference due to intranasal dropwise administration of the vaccine composition.

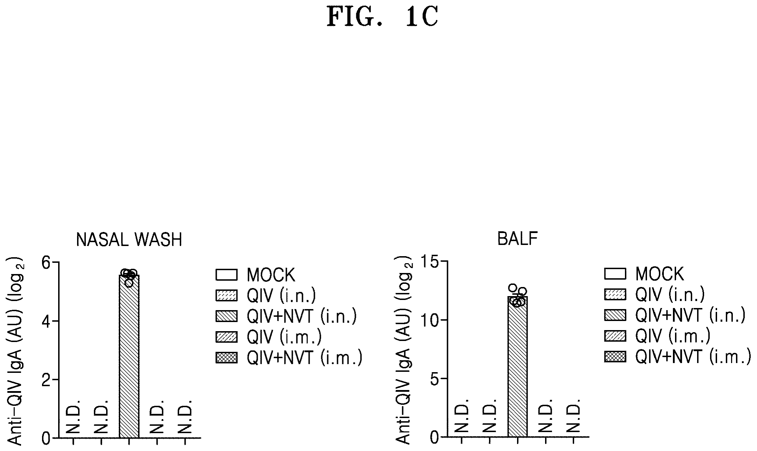

FIG. 1C is a diagram showing the results of measuring IgA levels in BALF, nasal wash, and serum after intramuscular and intranasal instillation, respectively, of a vaccine containing Teratect prefilled syringe (Influenza split vaccine), or NVT and Teratect prefilled syringe (Influenza split vaccine). As shown in FIG. 1C, IgA levels in BALF and nasal wash fluid were measured at higher levels compared to the control group. This indicates that intranasal administration of the vaccine composition increases IgA levels. In other words, it shows that the vaccine composition increases IgA levels through mucosal immunization.

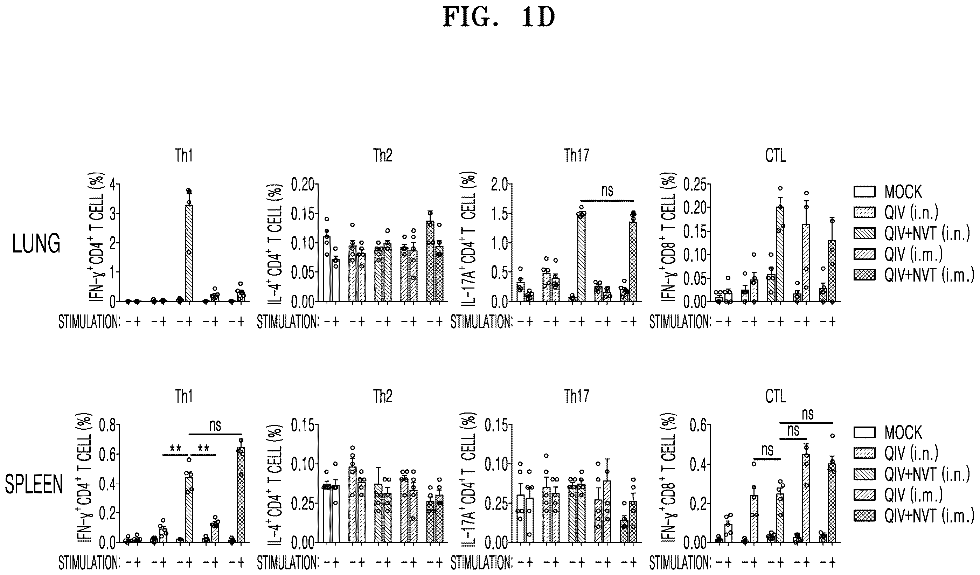

FIG. 1D shows the results of measuring antigen-specific T cell responses in the lung and spleen using intracellular cytokine staining, after intramuscular or intranasal dropwise administration of vaccines including Teratect prefilled syringe (Influenza split vaccine), or NVT and Teratect prefilled syringe (Influenza split vaccine). Compared to the control group, IFNγ+CD4+ T cells in the spleen increased in the group administered with the vaccine including NVT and Teratect prefilled syringe (Influenza split vaccine) antigen. Notably, IFNγ+CD4+ T cells in the lung were increased only when the vaccine including NVT and Teratect prefilled syringe (Influenza split vaccine) antigen was administered intranasally. In addition, it was confirmed that the CD4+ T cell response induced by the vaccine is a Th1-type CD4+ T cell response. Through this, it was confirmed that the IFNγ+CD4+ T cell response in the lung is induced only when the vaccine antigen is administered intranasally together with NVT.

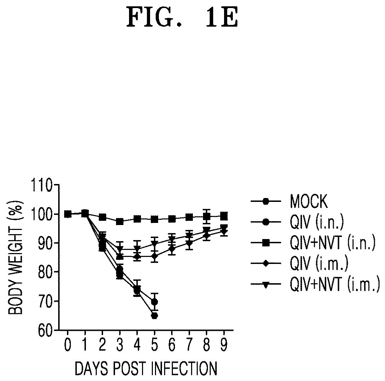

FIG. 1E is a diagram showing the results of measuring body weight over time after intramuscular or intranasal dropwise administration of vaccines containing Teratect prefilled syringe (Influenza split vaccine), or NVT and Teratect prefilled syringe (Influenza split vaccine). After administration of the designated amount of antigen, the same amount was administered once again as a booster dose two weeks later. Two weeks after the booster administration, Influenza A/Korea/2785/2009 (2009 pandemic strain), BALB/c adapted (provided by KCDC, National Culture Collection for Pathogens, NCCP43021), was challenge-inoculated intranasally at a dose of 5LD50. In FIG. 1, "QIV" represents Teratect prefilled syringe (Influenza split vaccine).

As shown in FIG. 1E, compared to the case where Teratect prefilled syringe (Influenza split vaccine) was administered intramuscularly alone, body weight was significantly better maintained when the vaccine including NVT and Teratect prefilled syringe (Influenza split vaccine) was administered intranasally. This suggests a correlation between the improved mucosal immune response and the potency of the vaccine.

### Example 2: Cross-Protection Verification Experiment

In this experiment, the H3N2 antigen from the commercially available Teratect prefilled syringe (Influenza split vaccine) was provided by the manufacturer and administered intramuscularly to subjects, or a vaccine mixed with NVT was prepared as described above and administered intranasally by dropwise application. Balb/c mice were used as the subjects.

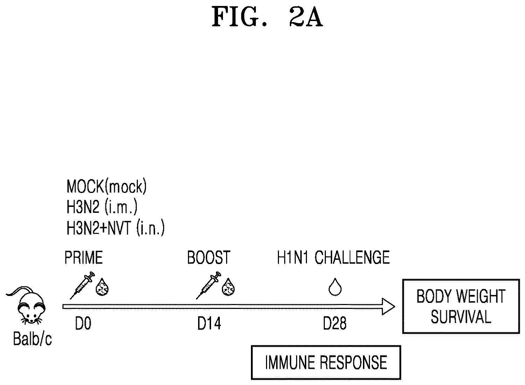

As shown in FIG. 2A, the vaccine was administered intranasally to the mice, and two weeks later, a booster dose was administered in the same manner. Two weeks after administration, BALF, nasal wash fluid, and serum were collected, and neutralizing antibody titers against each virus were measured by hemagglutination inhibition assay. In addition, antigen-specific T cell responses in the lung and spleen were measured using intracellular cytokine staining. In some mice, a challenge infection was performed two weeks after the booster administration by intranasal dropwise inoculation of a different subtype, H1N1 virus, at a dose of 3LD50.

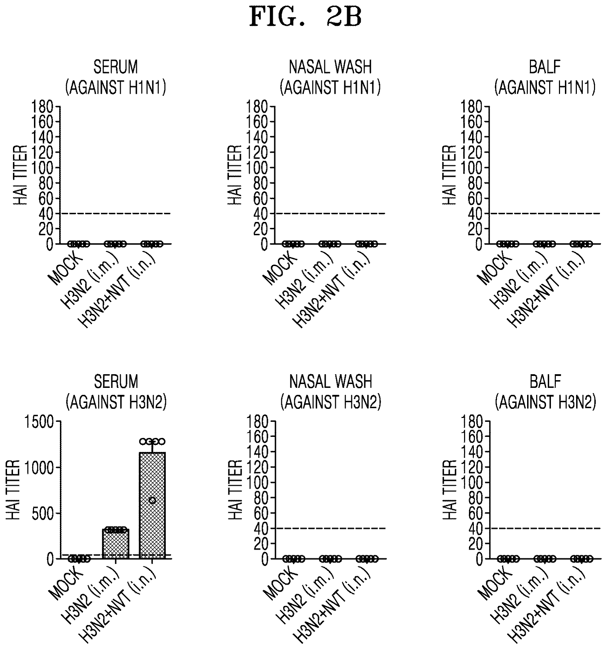

FIG. 2B shows the results of measuring neutralizing antibody responses against H3N2 (the same subtype as the vaccine) and H1N1 (a different subtype) in serum, BALF, and nasal wash fluid, after intramuscular or intranasal administration of vaccines including H3N2 antigen or NVT and H3N2 antigen, using the hemagglutination inhibition assay. In BALF and nasal wash fluid, neutralizing antibodies were not detected in any experimental group regardless of the administered vaccine. In serum, neutralizing antibodies were detected against the same subtype H3N2 antigen used in the vaccine, but not against the different subtype H1N1 virus. Through this, it was confirmed that the neutralizing antibodies observed in the serum do not show cross-reactivity.

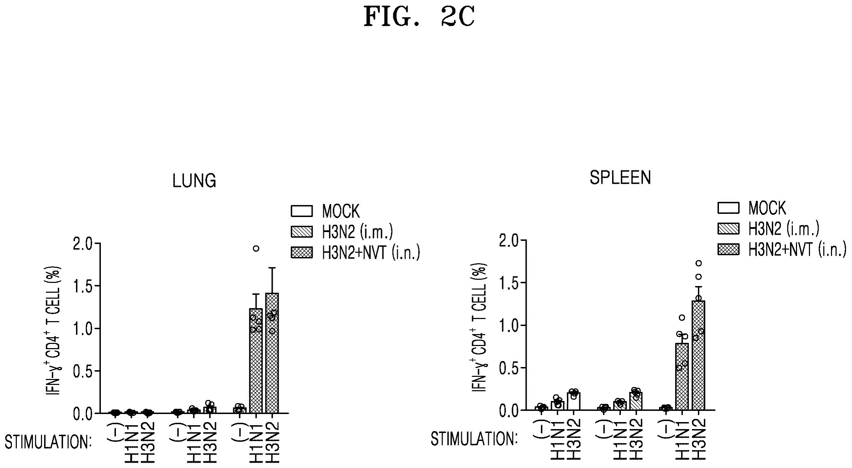

FIG. 2C is a diagram showing the results of measuring antigen-specific T cell responses in the lung and spleen against H3N2 (the same subtype as the administered vaccine) and H1N1 (a different subtype) using intracellular cytokine staining, after intramuscular or intranasal dropwise administration of vaccines comprising H3N2 antigen, or NVT and H3N2 antigen. As confirmed in the above-described FIG. 1D, antigen-specific CD4+ T cell responses were observed only in the lung and spleen when the vaccine composition included NVT. In particular, CD4+ T cell responses were observed not only against H3N2, the same subtype used in the vaccine, but also against H1N1, a different subtype from that used in the vaccine. This confirmed that, unlike neutralizing antibodies, CD4+ T cell responses may exhibit cross-reactivity to different subtypes.

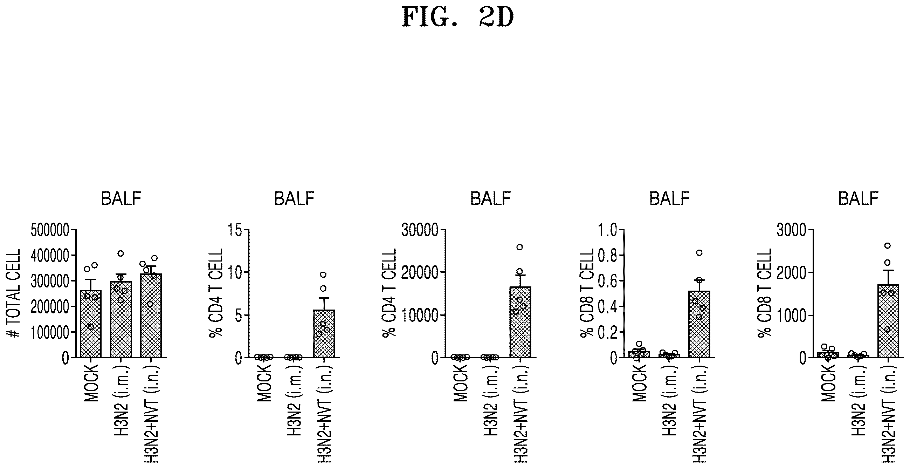

FIG. 2D shows the results of measuring the number of CD4+ T cells and CD8+ T cells in BALF after intramuscular or intranasal dropwise administration of vaccines including H3N2 antigen, or NVT and H3N2 antigen.

The total number of cells measured in BALF did not show significant differences compared to the control group in any of the experimental groups. However, CD4+ T cells and CD8+ T cells in BALF were detected only in the group that received the vaccine including NVT and H3N2 antigen via intranasal administration. Based on this result, it was confirmed that cell-mediated immune responses in the lower respiratory tract were formed only in the group administered intranasally with NVT and H3N2 antigen.

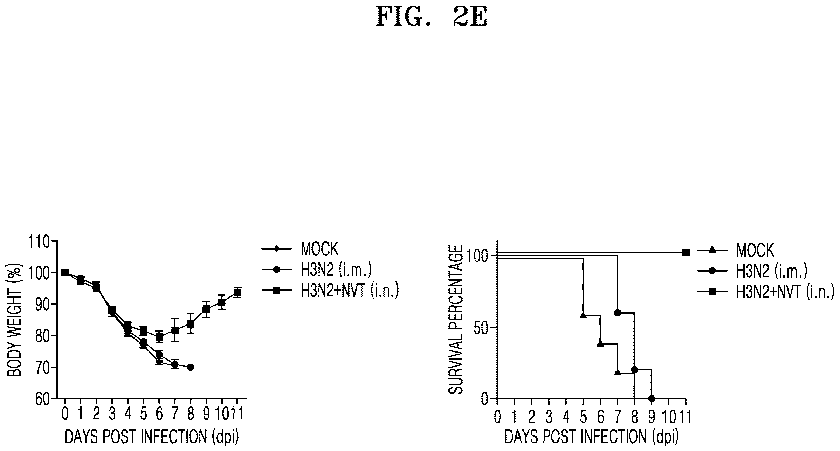

FIG. 2E is a diagram showing body weight changes and survival rate after challenge with an H1N1 virus, a different subtype from the antigen used in the vaccine, in mice administered with H3N2 antigen or a vaccine including NVT and H3N2 antigen via intramuscular or intranasal dropwise administration. As shown in FIG. 2E, body weight was higher in the group that received intranasal dropwise administration of the vaccine including NVT and H3N2 antigen, compared to the group that received H3N2 antigen via intramuscular injection. In addition, on day 6 after the challenge, the survival rate following challenge with a different subtype H1N1 virus was 0 % in the group administered with H3N2 antigen via intramuscular injection, and 100 % in the group administered with the vaccine including NVT and H3N2 antigen via intranasal administration. This indicates that when influenza A virus H3N2 antigen is administered in combination with NVT, the subject acquires protective capability against influenza A virus H1N1. This also indicates that when influenza A virus H1N1 antigen is administered in combination with NVT, the subject acquires protective capability against influenza A virus H3N2. In other words, administration of influenza A virus antigens in combination with NVT shows that the subject acquires protective capability against different subtypes of influenza A virus.

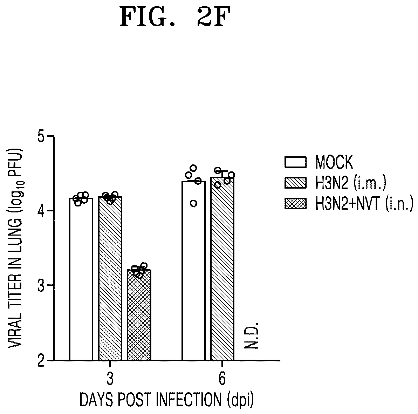

FIG. 2F is a diagram showing the viral titer in the lungs on day 3 and day 6 after challenge with an H1N1 virus, a different subtype from the antigen used in the vaccine, in mice that were administered H3N2 antigen or a vaccine comprising NVT and H3N2 antigen via intramuscular or intranasal dropwise administration. Only in the experimental group that received the vaccine including NVT and H3N2 antigen via intranasal administration, the viral titer in the lungs decreased tenfold on day 3 after the challenge. In addition, on day 6 after the challenge, no virus was detected in the lungs of that group. Through this, it was confirmed that in the group administered intranasally with NVT and H3N2 antigen, influenza virus replication in the lungs was suppressed.

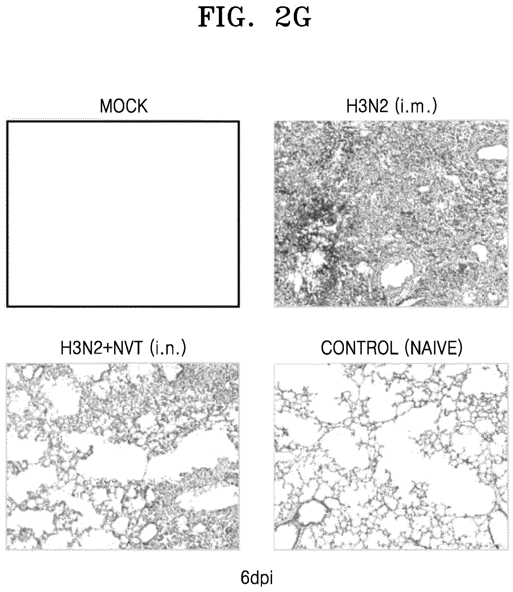

FIG. 2G shows the results of histopathological examination of the lungs on day 6 after challenge with an H1N1 virus, a different subtype from the antigen used in the vaccine, in mice that were administered H3N2 antigen or a vaccine including NVT and H3N2 antigen via intramuscular or intranasal dropwise administration. In the control group and the group administered H3N2 antigen via intramuscular injection, many inflammatory cells were observed to have infiltrated the lungs. However, in the group administered the vaccine including NVT and H3N2 antigen, the inflammatory state was found to be significantly alleviated.

### Example 3: Comparison of Reactivity and Protective Efficacy after In Vivo Depletion of CD4 T Cells

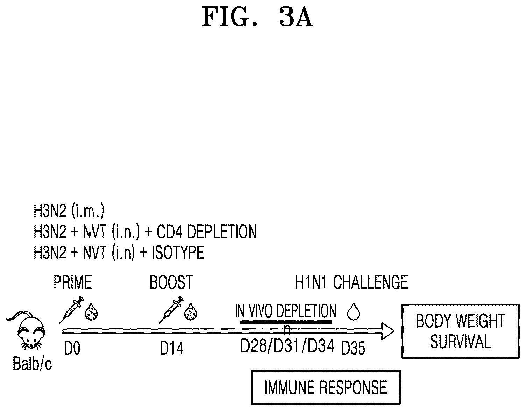

As shown in FIG. 3A, the vaccine was administered intranasally by dropwise application to mice, and two weeks later, a booster was administered in the same manner. Two weeks after the booster administration, a CD4 T cell depletion antibody (GK1.5 clone, BE0003-1, BioXcell) was administered intraperitoneally at 200 µg per injection on days 28, 31, and 34 after the initial immunization to deplete CD4 T cells in the subjects. On day 35 after the initial immunization, nasal wash fluid and serum were collected. Antigen-specific IgA was measured in the nasal wash fluid, and neutralizing antibody titers against each virus were measured using the hemagglutination inhibition assay. In addition, the number of CD4+ T cells in the lung and spleen was measured to confirm the presence or absence of residual CD4+ T cells. Some mice were challenged by intranasal dropwise administration of 3LD50 of H1N1 virus, a different subtype from the vaccine antigen.

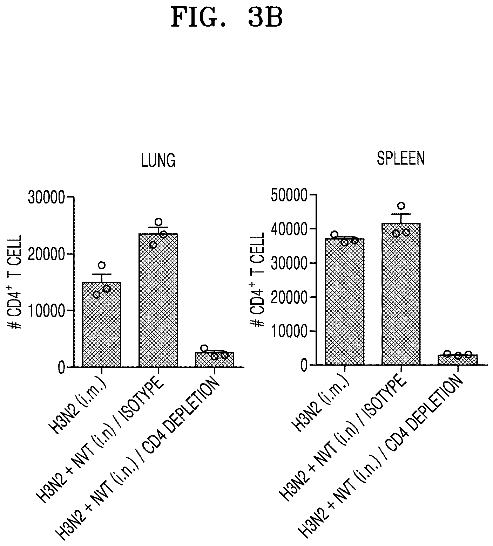

FIG. 3B shows the results of measuring the number of CD4+ T cells in the lung and spleen in the group administered H3N2 intramuscular vaccine or NVT + H3N2 intranasal vaccine followed by CD4 depletion antibody, and in the experimental group administered NVT + H3N2 intranasal vaccine followed by an isotype control antibody. In mice immunized with NVT and an H3N2 antigen-containing vaccine and subjected to CD4 T cell depletion, the levels of CD4+ T cells in the lung and spleen were significantly decreased compared to the control group upon challenge with H1N1 virus. In contrast, in the control groups, i.e., mice receiving H3N2 monovalent vaccine via intramuscular injection, or NVT and H3N2 antigen-containing vaccine via intranasal dropwise administration without CD4 T cell depletion but with isotype control antibody injection, the levels of CD4+ T cells in the lung and spleen were high. Through this, it was confirmed that CD4 depletion occurred successfully.

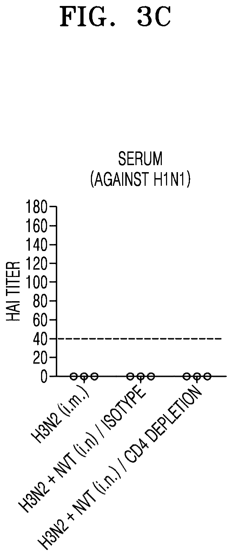

FIG. 3C shows the results of measuring neutralizing antibody titers against H1N1 (a different subtype from the vaccine antigen) in the serum of groups administered H3N2 intramuscular vaccine or NVT + H3N2 intranasal vaccine followed by CD4 depletion antibody, and the experimental group administered NVT + H3N2 intranasal vaccine followed by an isotype control antibody. Neutralizing antibodies against H1N1 were not detected in any group, confirming that CD4 depletion had no effect on the neutralizing antibody titers in the serum.

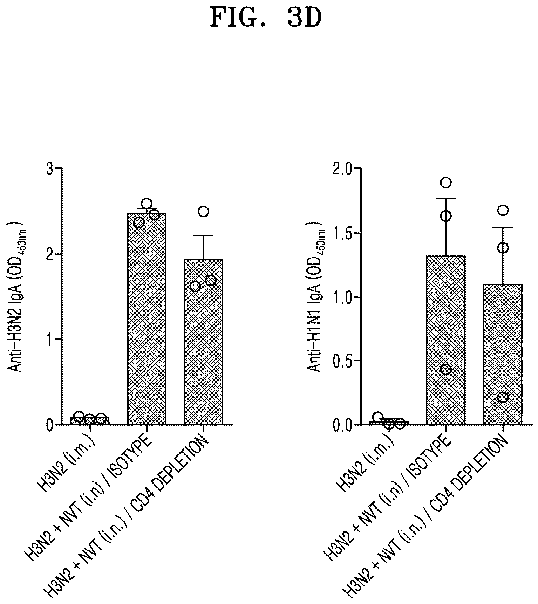

FIG. 3D shows the results of measuring antigen-specific IgA titers against H3N2 (same subtype as the vaccine antigen) and H1N1 (a different subtype) in nasal wash fluid of groups administered H3N2 intramuscular vaccine or NVT + H3N2 intranasal vaccine followed by CD4 depletion antibody, and the experimental group administered NVT + H3N2 intranasal vaccine followed by an isotype control antibody. Regardless of CD4 depletion, IgA was detected in all experimental groups that received intranasal vaccination, confirming that CD4 depletion had no effect on IgA production.

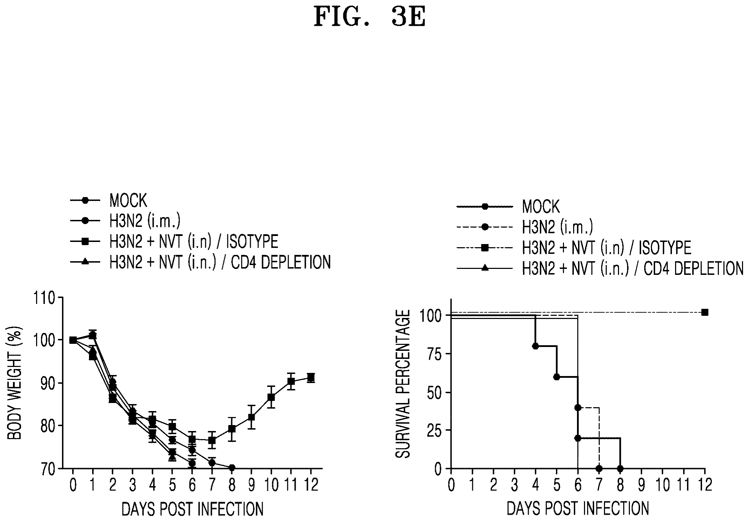

FIG. 3E is a diagram showing body weight changes and survival rate over time in mice challenged with H1N1 virus in groups administered H3N2 intramuscular vaccine or NVT and H3N2 antigen-containing vaccine intranasally, with or without CD4 T cell depletion. As confirmed in the previous figure (FIG. 3D), no cross-protection against H1N1 was observed in the group that received intramuscular H3N2 vaccine, whereas cross-protection was observed in the group that received NVT and H3N2 intranasally. However, when CD4 T cells were depleted even after intranasal administration of NVT and H3N2, cross-protection was not observed, confirming that the CD4 T cell response is a key mechanism contributing to cross-protection.

### Example 4: Comparison of Protective Effects According to Lung-localized CD4 Trm Cells Based on Administration Route

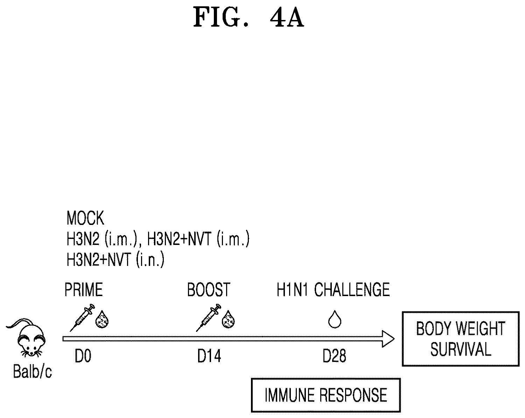

As shown in FIG. 4A, the vaccine was administered to mice by intranasal dropwise application, and two weeks later, a booster dose was administered using the same method. Two weeks after administration, antigen-specific T cell responses in the lung and spleen were measured using intracellular cytokine staining. In addition, lung resident memory CD4 T cells (CD62L-CD44+CD69+CD4+) formed in the lung were measured. In some mice, a challenge infection with H1N1 virus, a different subtype, was conducted via intranasal dropwise administration of 3LD50 two weeks after the booster dose.

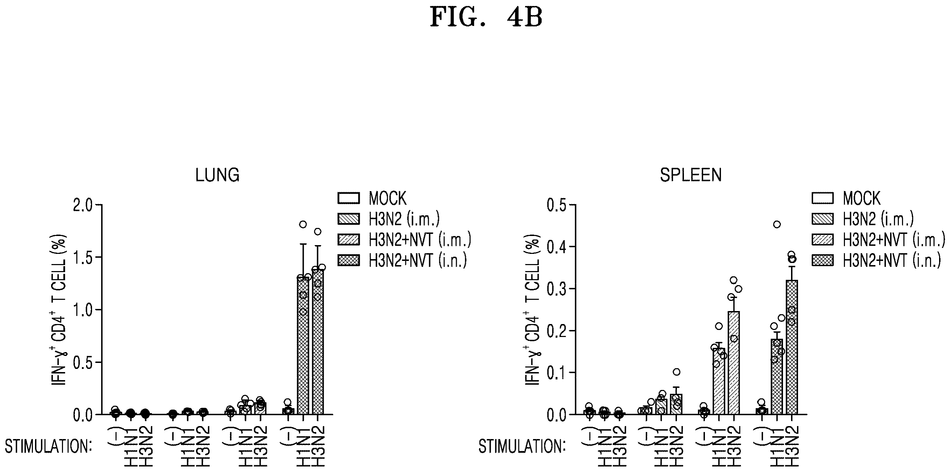

FIG. 4B is a diagram comparing antigen-specific T cell responses observed in the lung and spleen after administration of H3N2 intramuscular vaccine, NVT+H3N2 intranasal vaccine, or NVT+H3N2 intramuscular vaccine. Among all experimental groups, T cell responses were detected only in the groups that included NVT. Among them, T cell responses in the lung were confirmed only in the group that received NVT+H3N2 via intranasal administration. Through this, it was confirmed that even if NVT is included in the vaccine composition, the induction of T cell responses in the lung is determined by the route of administration.

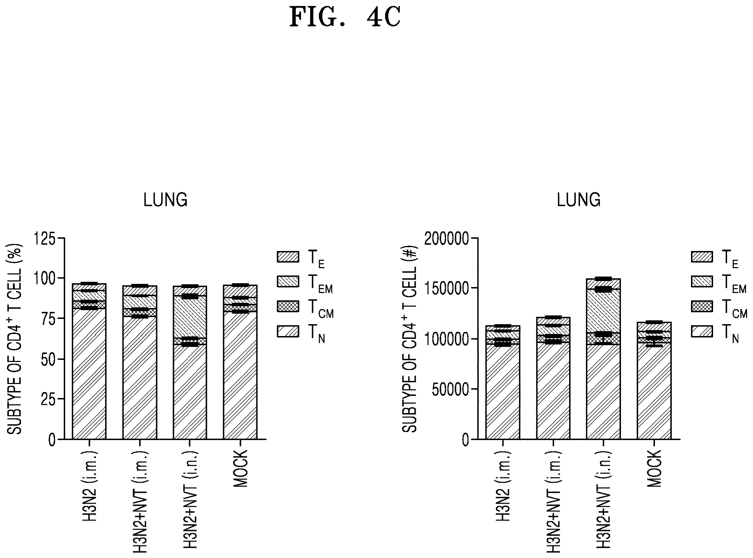

FIG. 4C is a diagram comparing T cell subsets in the lung after administration of H3N2 intramuscular vaccine, NVT+H3N2 intranasal vaccine, or NVT+H3N2 intramuscular vaccine. It was confirmed that the proportion and number of effector memory T cells in the lung increased only in the experimental group administered NVT+H3N2 intranasally.

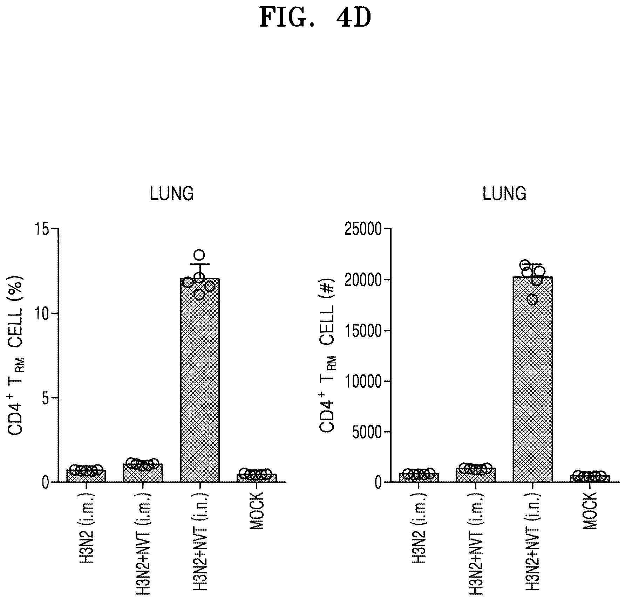

FIG. 4D is a diagram comparing the proportion and number of resident memory T cell subsets in the lung after administration of H3N2 intramuscular vaccine, NVT + H3N2 intranasal vaccine, or NVT + H3N2 intramuscular vaccine. It was confirmed that the proportion and number of resident memory T cells in the lung increased only in the experimental group administered NVT + H3N2 intranasally.

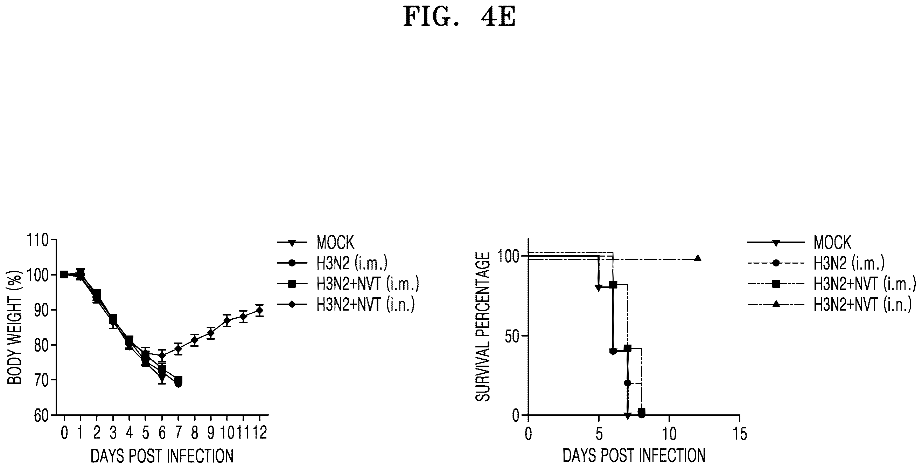

FIG. 4E is a diagram showing body weight changes and survival rate over time after challenge with H1N1 virus in mice administered H3N2 intramuscular vaccine, NVT + H3N2 intranasal vaccine, or NVT + H3N2 intramuscular vaccine. Cross-protection was not observed in the mice administered H3N2 intramuscular vaccine or NVT + H3N2 intramuscular vaccine, whereas cross-protection was observed only in the experimental group administered NVT + H3N2 intranasally. Through this, it was confirmed that the survival rate after challenge is closely related to the formation of resident memory T cells in the lung.

### Example 5: Trm Formation Mechanism

To confirm the mechanism by which Trm is formed by the NVT + H3N2 intranasal vaccine, in this experiment, the H3N2 antigen from the commercially available Teratect prefilled syringe (Influenza split vaccine) was provided by the manufacturer, and a vaccine mixed with NVT was prepared and administered intranasally by dropwise application to the subject. C57BL/6 mice and IFN-αR1 knockout (KO) mice of the same strain were used as the subjects.

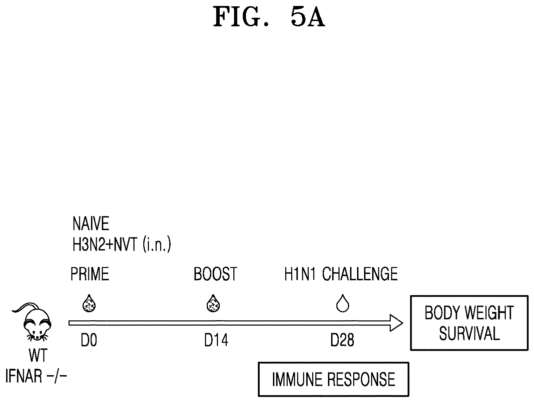

As shown in FIG. 5A, the vaccine was administered intranasally by dropwise application to the mice, and two weeks later, a booster dose was administered using the same method. Two weeks after administration, nasal wash fluid and serum were collected. Using the ELISA method, the levels of antigen-specific IgG antibodies in the serum were measured, and the levels of antigen-specific IgA antibodies in the nasal wash fluid were measured. Antigen-specific T cell responses in the lung and spleen were measured using intracellular cytokine staining. In addition, lung resident memory CD4 T cells (CD62L-CD44+CD69+CD4+) formed in the lung were measured.

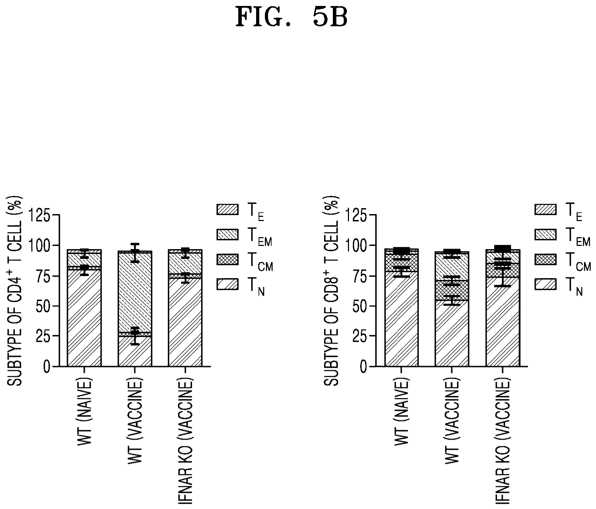

FIG. 5B is a diagram comparing T cell subsets in the lung after administration of NVT + H3N2 intranasal vaccine to WT (wild type) mice and KO (knockout) mice, respectively. It was confirmed that the proportion and number of effector T cells in the lung increased only in WT mice.

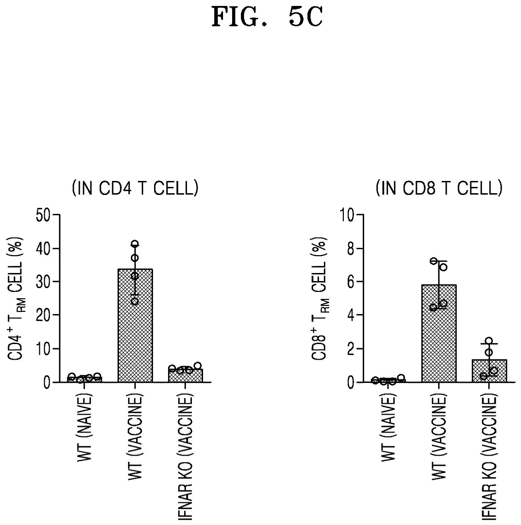

FIG. 5C is a diagram comparing the proportion and number of resident memory T cell subsets in the lung after administration of NVT + H3N2 intranasal vaccine to WT (wild type) mice and KO (knockout) mice, respectively. In WT mice, the proportion and number of resident memory T cells in the lung were increased, whereas in KO mice, resident memory T cells were not formed in the lung. Through this, it was confirmed that type I interferon is involved in the formation of resident memory T cells in the lung.

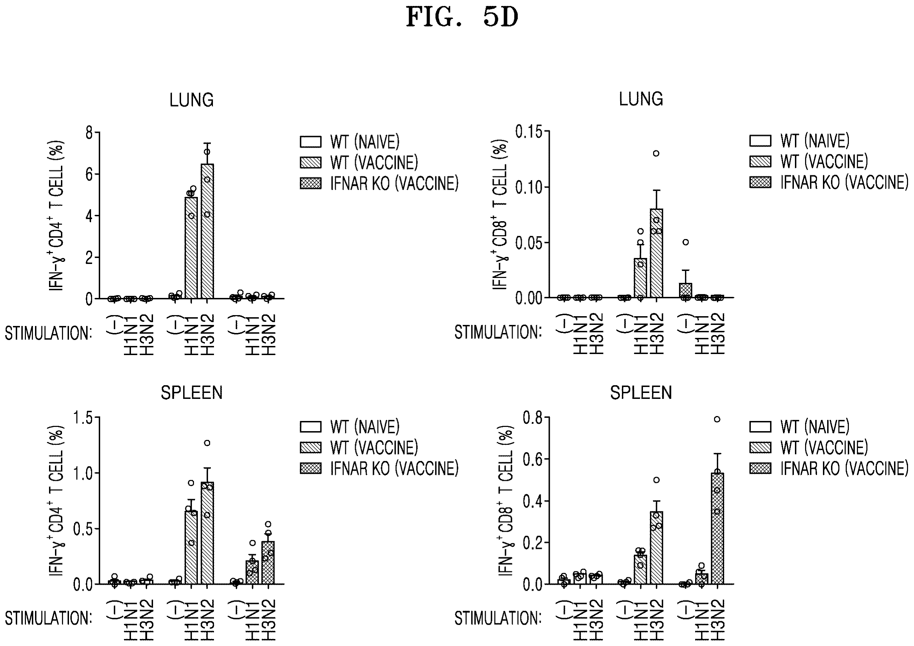

FIG. 5D is a diagram comparing antigen-specific T cell responses in the lung and spleen after administration of NVT + H3N2 intranasal vaccine to WT (wild type) mice and KO (knockout) mice, respectively. In the spleen, antigen-specific T cell responses were observed in all experimental groups except the negative control (naive). However, antigen-specific T cell responses in the lung were observed only in WT mice and were not observed at all in KO mice. This confirmed that type I interferon is involved in T cell responses in the lung.

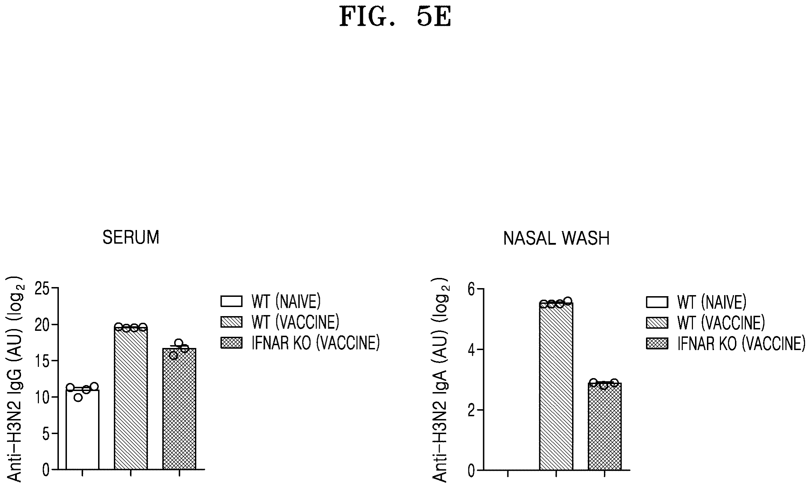

FIG. 5E is a diagram comparing antigen-specific IgG and IgA titers in the serum and nasal wash fluid after administration of NVT + H3N2 intranasal vaccine to WT (wild type) mice and KO (knockout) mice, respectively. Antigen-specific IgG and IgA titers were detected in both WT and KO mice, although there were slight quantitative differences. Through this, it was confirmed that type I interferon has a limited role in NVT-mediated antibody production.

### Example 6: Comparison of Cross-Protection Between Different Antigens

Influenza H1N1 strains may be classified into pdm09 strains and non-pdm09 strains based on the 2009 pandemic, and cross-protection does not occur between pdm09 and non-pdm09 strains.

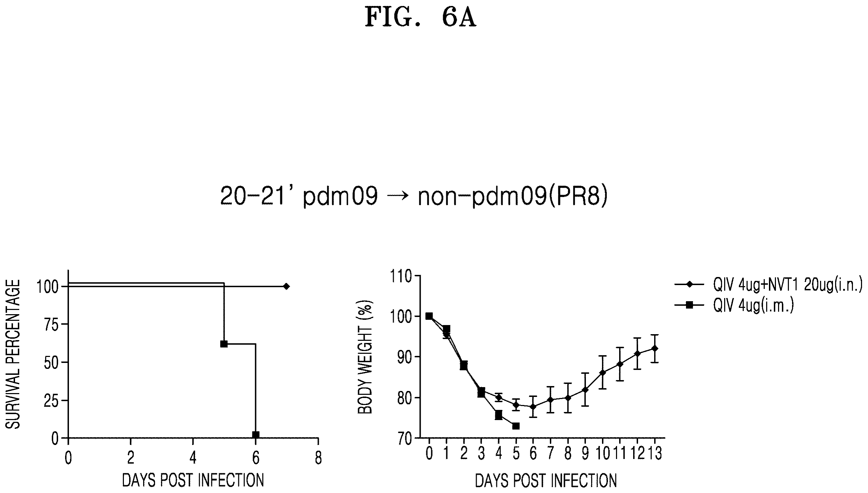

FIG. 6A is a graph showing survival rate and body weight changes after two intramuscular administrations at two-week intervals of a quadrivalent vaccine (QIV) containing the H1N1 pdm09 strain, or intranasal administration in combination with NVT, followed by challenge with the H1N1 non-pdm09 strain, influenza A/PR8/34 (H1N1). In the group administered QIV intramuscularly, all mice died upon PR8 challenge. In contrast, in the group administered intranasally with QIV and NVT, all mice were protected from PR8 infection. Through this, it was confirmed that cross-protection may occur even between the pdm09 and non-pdm09 strains.

Type A influenza may also be classified according to host species. In the case of H3N2 strains, human H3N2 and canine H3N2 exist separately, and cross-protection does not occur between these two strains.

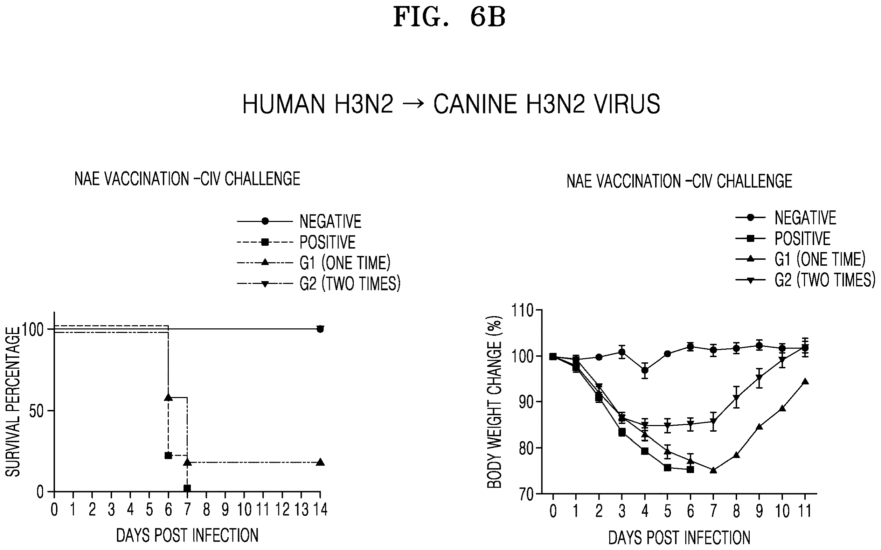

FIG. 6B shows graphs of survival rate and body weight changes after two intramuscular administrations at two-week intervals of a human H3N2 vaccine, or intranasal administration in combination with NVT, followed by challenge with a canine H3N2 strain. When human H3N2 was administered intramuscularly, all mice died when challenged with canine H3N2, whereas when NVT was administered intranasally together with NVT, all mice were protected from canine H3N2 infection. Through this, it was confirmed that cross-protection occurred between human H3N2 strains and canine H3N2 strains when the vaccine was administered intranasally using NVT.

The commercially available Teratect prefilled syringe (Influenza split vaccine) is a quadrivalent antigen vaccine containing Influenza A H1N1 and H3N2, and Influenza B BV and BY antigens. The B influenza viruses BV and BY correspond to the Victoria lineage and Yamagata lineage, respectively. The reason for using a quadrivalent vaccine is that each of the four viruses does not provide cross-protection against the others, even when each antigen is administered individually.

In this experiment, the BV antigen from the commercially available Teratect prefilled syringe (Influenza split vaccine) was provided by the manufacturer and administered intramuscularly to subjects; the BY antigen from the same vaccine was also provided and administered intramuscularly to subjects; or a vaccine comprising BY antigen mixed with NVT was prepared and administered intranasally by dropwise application. The BV virus used in the vaccine is the B/Maryland/15/2016 strain. The BY virus used in the vaccine is the B/Phuket/3073/2013 strain.

Balb/c mice were used as the subjects. The antigen was administered at a dose of 2 µg, followed by a booster dose of the same amount two weeks later. Two weeks after the booster, a challenge infection was performed by intranasal dropwise administration of 3LD50 of a different subtype BV virus, B/Shandong/7/97.

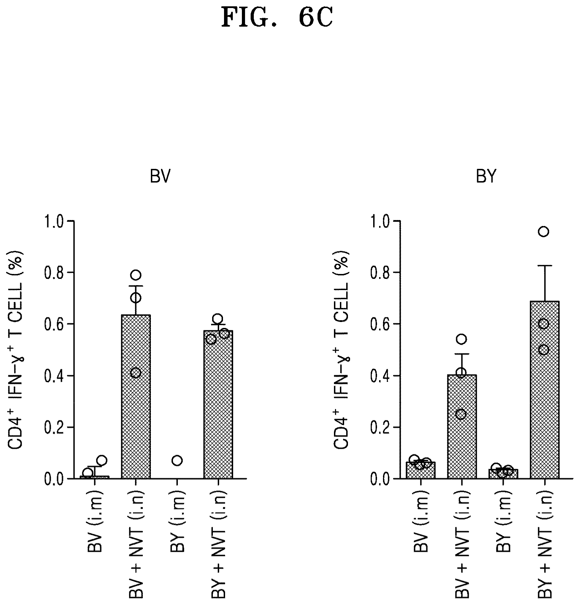

As shown in FIG. 6C, when stimulated with BV virus, high levels of CD4+ IFNγ+ T cells were measured in both groups that received intranasal dropwise administration of inactivated BV and NVT-containing vaccine, and inactivated BY and NVT-containing vaccine. In contrast, low levels of CD4+ IFNγ+ T cells were measured in both groups that received intramuscular administration of inactivated BV-containing vaccine and inactivated BY-containing vaccine.

In addition, when stimulated with the BY virus, high levels of CD4+ IFNv+ T cells were measured in both groups that received intranasal dropwise administration of inactivated BV and NVT-containing vaccine, and inactivated BY and NVT-containing vaccine.. In contrast, low levels of CD4⁺ IFNγ⁺ T cells were measured in both groups that received intramuscular administration of inactivated BV-containing vaccine and inactivated BY-containing vaccine.

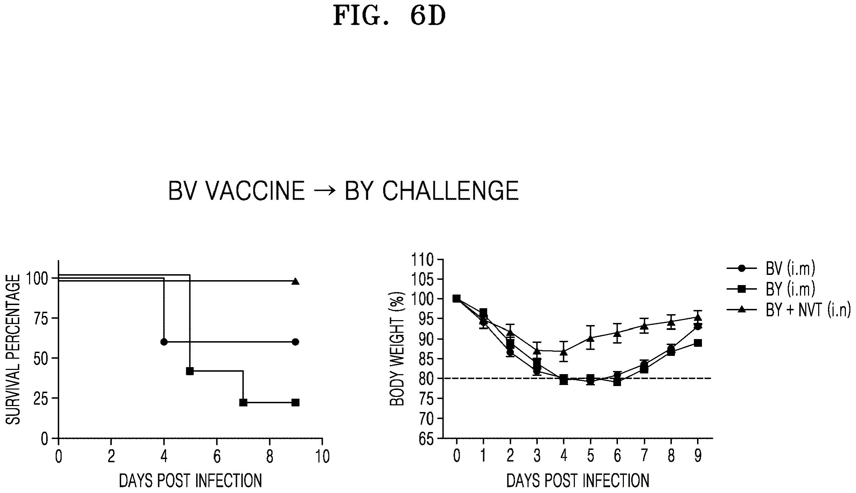

FIG. 6D shows the results of measuring body weight over time following intramuscular or intranasal administration of vaccines including BV antigen, BY antigen, or NVT and BY antigen, as well as survival rates after challenge with a different subtype BV virus. As shown in FIG. 6D, compared to intramuscular injection of BY or BV antigen alone, the group that received intranasal administration of the vaccine including NVT and BY antigen showed a higher survival rate following challenge with the different subtype BV virus.

This indicates that when the influenza B virus BY antigen is administered in combination with NVT, the subject acquires protective capability against influenza B virus BV. It also indicates that when the influenza B virus BV antigen is administered in combination with NVT, the subject acquires protective capability against influenza B virus BY. In other words, administration of influenza B virus antigens in combination with NVT enables the subject to acquire protective capability against different subtypes of influenza B virus. This demonstrates that intranasal dropwise administration of inactivated BV and NVT-containing vaccine, or inactivated BY and NVT-containing vaccine, may provide cross-reactivity against different subtypes of the same B virus.

Therefore, intranasal dropwise administration of a vaccine including an inactivated antigen of a specific type A, B, or C virus and NVT may provide cross-reactivity against other subtypes of the same type A, B, or C virus. This indicates that intranasal dropwise administration of a vaccine including an inactivated antigen of a specific type of influenza virus and NVT may provide protective immunity even against different types of influenza virus, thereby offering protection against all types of influenza virus regardless of the type. In other words, it suggests that such a formulation may be used as a universal influenza vaccine.

### Example 7: Comparison of Neutralizing Antibody Responses and T cell Reactivity Between Different Subtype/Substrain antigens

Since the strains of influenza virus that become prevalent change every year, vaccines against influenza virus use antigens derived from different influenza virus strains each year.

In this experiment, it was examined whether a vaccine for an influenza virus that was prevalent in a particular year could provide protection against an influenza virus that became prevalent in a different year. Table 1 below shows the influenza viruses that were prevalent by year.

**[Table 1]**

| Type | Year | Virus |
|---|---|---|
| H1N1 | 20-21 | 1.A/Guangdong-Maonan/SWL 1536/2019 |
| | 19-20 | 2.A/Brisbane/02/2018 |
| | 18-19 | 3.A/Michigan/45/2015 |
| H3N2 | 20-21 | 4.A/Hong Kong/2671/2019 |
| | 19-20 | 5.A/Kansas/14 |
| | 18-19 | 6.A/Singapore/INFIMH-16-0019/2016 |
| BY | 18-21 | 7.B/Phuket/3073/3/2013 |
| | 20-21 | 8.B/Victoria/705/2018 |
| BV | 19-20 | 9.B/Maryland/15/2016 |
| | 18-19 | 10.B/Brisbane/60/2008 |

Specifically, the commercially available quadrivalent vaccine, Teratect prefilled syringe (Influenza split vaccine), was administered intramuscularly to subjects, or a vaccine was prepared by mixing Teratect prefilled syringe (Influenza split vaccine) with NVT and administered intranasally by dropwise application. The Teratect prefilled syringe (Influenza split vaccine) is a quadrivalent antigen vaccine containing A influenza H1N1 and H3N2, and B influenza BV and BY antigens, which includes antigens derived from strains 1, 4, 7, and 8 of the viruses that were prevalent in 2020-2021, as listed in Table 1. Balb/c mice were used as the subjects. The designated amount of antigen was administered, followed by a booster dose of the same amount two weeks later. Two weeks after the booster administration, serum was isolated from the subjects, and the HAI titer was measured using the hemagglutination inhibition method to assess neutralizing antibody levels. In addition, two weeks after the challenge, lungs were isolated from the subjects, and the level of CD4+ IFNv+ T cells in the lungs was measured. The measurement of lung CD4+ IFNv+ T cells was performed using intracellular cytokine staining.

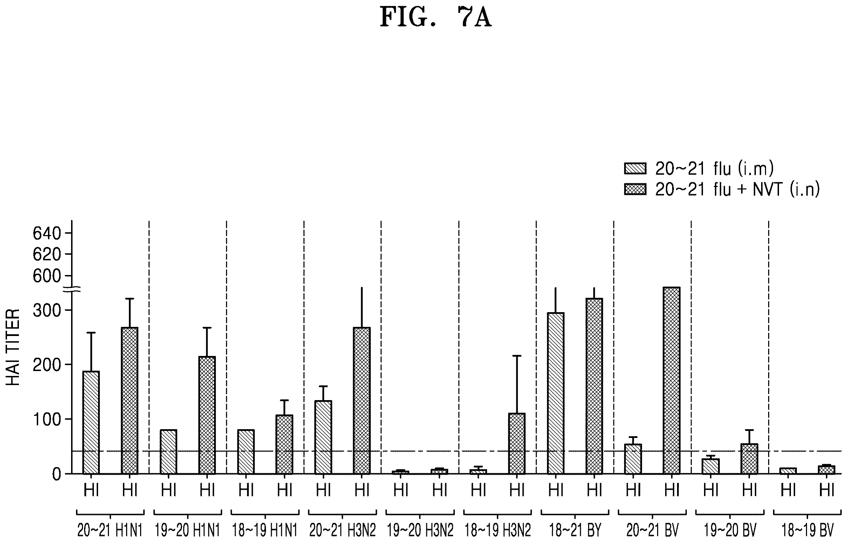

FIG. 7A shows the results of HAI titer measurements after intramuscular or intranasal dropwise administration of the Teratect prefilled syringe (Influenza split vaccine), or a vaccine containing NVT and Teratect prefilled syringe (Influenza split vaccine). As shown in FIG. 7A, neutralizing antibody production was not induced for some viruses.

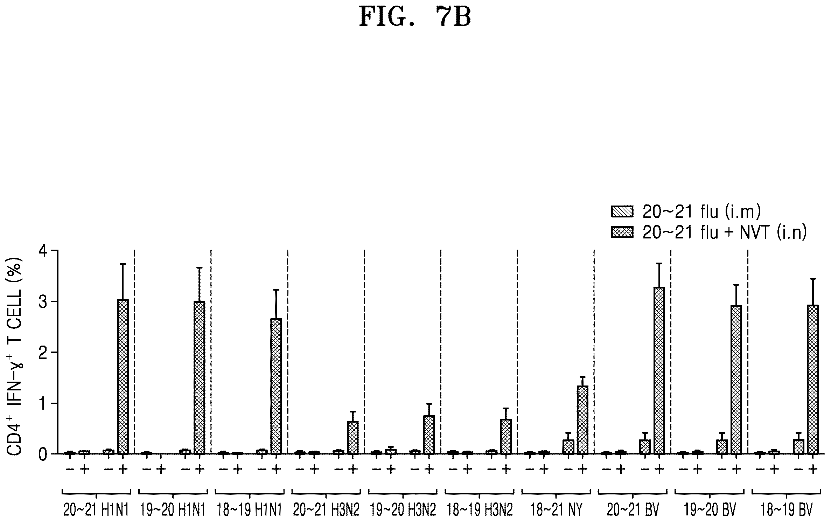

FIG. 7B shows the results of measuring CD4+ IFNv+ T cell levels in the lung after intramuscular or intranasal dropwise administration of the Teratect prefilled syringe (Influenza split vaccine), or a vaccine containing NVT and Teratect prefilled syringe (Influenza split vaccine). As shown in FIG. 7B, when the vaccine containing NVT and Teratect prefilled syringe (Influenza split vaccine) was administered via the mucosal route, T cell production was increased.

As described above, when the antigen-containing vaccine including NVT and influenza virus antigen was administered via the mucosal route, antigen-specific T cell responses were induced in the respiratory tract, including the mucosa and/or the lungs, as well as in the spleen. Unlike the antibody response, this T cell response exhibited cross-reactivity to other antigens, such as variant viruses. Accordingly, the antigen-containing vaccine including NVT and an influenza virus antigen may enhance not only protection against the administered antigen, for example, a specific virus strain administered, but also protection against its variants or other types of viruses.

### Example 8: Pulmonary Delivery According to Dropwised Volume

In intranasal dropwise administration to mice, the extent of pulmonary delivery varies depending on the volume of dropwise administration. To verify this, 10 µL and 30 µL of Evans blue solution were dropwise administered to the nostrils of mice, and after dropwise administration, the lungs were isolated to compare the extent of delivery.

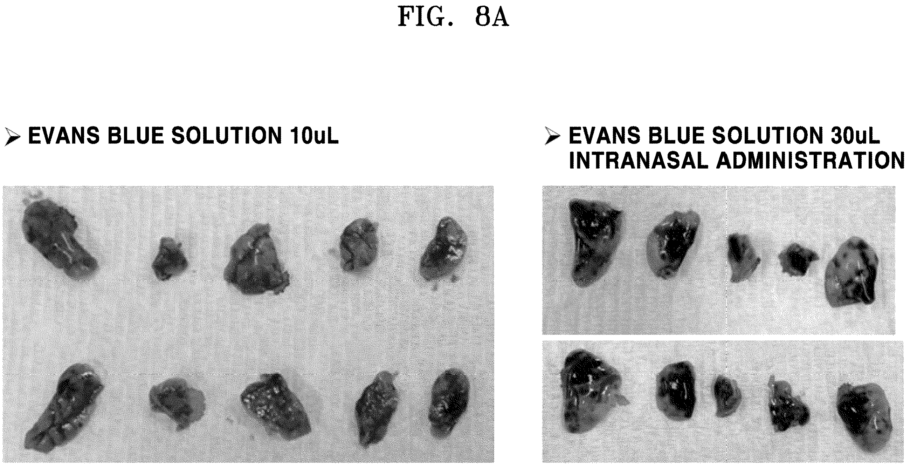

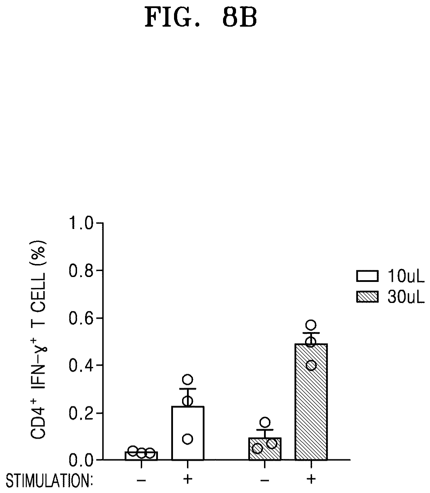

As seen in FIG. 8A, when 10 µL was administered, no delivery to the lungs was observed, whereas delivery to the lungs was confirmed with the 30 µL administration. As shown in FIG. 8B, it was confirmed that when the drug was delivered to the lungs, cellular immune responses in the lungs were effectively induced.

## Claims

1. A pulmonary delivery vaccine composition for preventing or treating influenza virus infection, comprising an influenza virus antigen as an active ingredient,
wherein the influenza virus antigen comprises hemagglutinin (HA) and nucleocapsid protein (NP), and
the vaccine composition comprises hetero-structured RNA (hsRNA), and is delivered to lungs.

2. The vaccine composition of claim 1, wherein the hetero-structured RNA comprises:
a first strand comprising a 5'-end, a 3'-end, and a region complementary to a second strand,
the second strand comprising a 5'-end, a 3'-end, and a region complementary to the first strand,
a first overhang located at the 3'-end of the first strand and comprising one or more consecutive ribonucleotides, and
a second overhang located at the 3'-end of the second strand and comprising one or more consecutive ribonucleotides.

3. The vaccine composition of claim 2, wherein the regions having complementarity in the first strand and the second strand are independently derived from a non-human organism.

4. The vaccine composition of claim 2, wherein the first strand and the second strand have the nucleotide sequences of SEQ ID NO: 1 and SEQ ID NO: 2, respectively.

5. The vaccine composition of claim 1, wherein the influenza virus antigen is one or more selected from the group consisting of influenza type A antigens and type B antigens.

6. The vaccine composition of claim 1, wherein the influenza virus antigen is an inactivated or live-attenuated whole influenza virus, subvirion, or subunit.

7. The vaccine composition of claim 1, providing heterosubtypic immunity and homosubtypic immunity against the influenza virus.

8. The vaccine composition of claim 1, wherein the composition is administered via a respiratory tract.

9. The vaccine composition of claim 1, wherein the vaccine composition induces a neutralizing antibody response and a T cell immune response.

10. The vaccine composition of claim 9, wherein the T cell immune response is a CD4+ T cell immune response.

11. The vaccine composition of claim 1, wherein type I interferon (IFN) is induced by the hetero-structured RNA delivered to the lungs, resulting in formation of CD4+ tissue-resident memory T (Trm) cells in the lungs.

12. The vaccine composition of claim 1, wherein the vaccine composition induces both humoral and cellular immune responses, resulting in cross-protection.
